# EUROPÄISCHE PATENTSCHRIFT

(11) **EP 3 402 910 B1**
(45) Veröffentlichungstag und Bekanntmachung des Hinweises auf die Patenterteilung: **23.10.2019**
(21) Anmeldenummer: 17708976.0
(22) Anmeldetag: 13.01.2017
(51) Int. Cl.: A61L 31/08, C23C 16/505, C23C 16/02, C23C 16/26, A61L 31/14, C23C 16/56

(54) **VERFAHREN ZUM HERSTELLEN EINER DÜNNEN SCHICHT AUS PORÖSEM DLC, VERWENDUNG EINER PECVD-ANLAGE UND MIT PORÖSEM DLC BESCHICHTETES WERKSTÜCK**
PROCESS FOR PRODUCING A THIN LAYER OF POROUS DLC, USE OF A PECVD PLANT AND WORKPIECE COATED WITH POROUS DLC
PROCÉDÉ DE PRODUCTION D'UNE COUCHE MINCE EN DLC POREUX, UTILISATION D'UNE INSTALLATION PECVD ET PIÈCE REVÊTUE DUDIT DLC POREUX

(30) Priorität: 14.01.2016 DE 102016200367
(43) Veröffentlichungstag der Anmeldung: 21.11.2018
(73) Patentinhaber: Hochschule Wismar, 23966 Wismar (DE)
(72) Erfinder: WIENECKE, Marion, 23966 Wismar (DE); SCHÜTZ, Antje, 23974 Stove (DE); HEEG, Jan, 18057 Rostock (DE)
(74) Vertreter: Seemann & Partner Patentanwälte mbB
(86) Internationale Anmeldenummer: PCT/EP2017/050733
(87) Internationale Veröffentlichungsnummer: WO 2017/121885

(56) Entgegenhaltungen:
- JP-A- 2009 186 236
- US-A- 5 939 149
- US-A1- 2005 260 411
- US-A1- 2015 104 648
- KOSKINEN J ET AL: "POROSITY OF THIN DIAMOND-LIKE CARBON FILMS DEPOSITED BY AN ARC DISCHARGE METHOD", SURFACE AND COATINGS TECHNOLOGY, ELSEVIER BV, AMSTERDAM, NL, Bd. 62, 1. Januar 1993 (1993-01-01), Seiten 356-360, XP000570610, ISSN: 0257-8972, DOI: 10.1016/0257-8972(93)90267-R

## Beschreibung

Die Erfindung betrifft ein Verfahren zum Herstellen einer dünnen Schicht aus porösem diamantähnlichem Kohlenstoff (DLC) mit plasmaunterstützter chemischer Gasphasenabscheidung (PECVD). Ferner betrifft die Erfindung die Verwendung einer PECVD-Anlage sowie ein Werkstück mit einer Oberfläche, die mit einer porösen DLC-Schicht beschichtet ist.

Mit Beschichtungen aus Kohlenstoff lassen sich vielfältige Oberflächenfunktionen realisieren. Solche Funktionsschichten sind dünne Schichten, die überwiegend aus Kohlenstoff bestehen. Beispielsweise handelt es sich um amorphe Kohlenstoffschichten, die in CVD-Verfahren (engl. chemical vapor deposition), also mittels chemischer Gasphasenabscheidung, hergestellt sind. Eine Form von Kohlenstofffunktionsschichten sind DLC-Schichten (engl. diamond like carbon).

DLC weist eine große Härte und einen hohen Verschleißwiderstand auf. In der Kraftfahrzeugtechnik werden DLC-Schichten beispielsweise im Verbrennungsmotor eingesetzt. Bewegliche Teile, wie etwa die Nockenwelle, die Kolbenringe oder auch Zahnräder, werden mit DLC beschichtet, um Verschleiß und Reibung zu minimieren und so die Fahrzeuge haltbarer, leistungsstärker und emissionsärmer zu machen. Außerdem kommen DLC-Schichten als Beschichtung auf Heiz- oder Brennelementen oder aufgrund ihrer guten Biokompatibilität als Oberflächenbeschichtung auf Implantaten zum Einsatz.

Eine Variante der chemischen Gasphasenabscheidung (CVD) ist die plasmaunterstützte chemische Gasphasenabscheidung (PECVD). Bei dieser wird die chemische Abscheidung durch ein Plasma unterstützt. Während der CVD erfolgt eine Dissoziation (Aufbrechen) der Moleküle eines Reaktions- oder Precursor-Gases durch externe Zufuhr von Wärme sowie durch die freiwerdende Energie der chemischen Reaktion. Bei der PECVD übernehmen diese Aufgabe die beschleunigten Elektronen im Plasma. Zusätzlich zu den so gebildeten Radikalen werden in einem Plasma Ionen erzeugt, die zusammen mit den Radikalen die Schichtabscheidung auf dem Substrat bzw. Werkstück bewirken. Die Gastemperatur im Plasma erhöht sich dabei in der Regel nur um wenige 100 °C, wodurch mit der PECVD im Gegensatz zur CVD auch temperaturempfindlichere Materialien problemlos beschichtet werden können.

Das Plasma kann direkt beim zu beschichtenden Substrat oder Werkstück oder in einer eigenen Kammer (Remote-Plasma-Methode) brennen. Bei der zuerst genannten sogenannten Direktplasmamethode wird zwischen dem zu beschichtenden Substrat und einer Gegenelektrode ein starkes elektrisches Feld angelegt, durch das ein Plasma gezündet wird. Ein direktes Plasma hat den Vorteil, dass eine in-situ Plasmasubstratreinigung durchgeführt werden kann.

Mit PECVD hergestellte DLC-Beschichtungen werden als Diffusionsbarriere auf Implantatwerkstoffen eingesetzt, um ein Ausdiffundieren von Metallionen zu verhindern. Die Beschichtung ist chemisch inert, haft- und verschleißfest.

Aus JP 2009-186236 A ist ein Werkstück aus einem lichtdurchlässigen Substrat bekannt, welches mit einer Beschichtung aus DLC versehen ist. Die DLC-Schicht wird durch Abscheidung mittels PECVD unter Verwendung von Acetylen hergestellt. Die DLC-Schicht ist porös, der Volumenanteil der Poren beträgt zwischen 40% und 70%.

Aus US 2005/0260411 A1 ist ein wasserstofffreier DLC-Film bekannt, der durch Aufwachsen und Zurückätzen hergestellt wird. Die DLC-Schicht ist porös, die Porosität liegt zwischen 15% und 60%.

Patentdokument US 5,939,149 offenbart eine DLC Schicht, die durch ein Rückätzverfahren modifiziert wird.

Es ist eine Aufgabe der Erfindung, ein Verfahren zum Herstellen einer dünnen Schicht aus DLC, die Verwendung einer PECVD-Anlage sowie ein Werkstück mit einer mit DLC-beschichteten Oberfläche anzugeben, wobei die DLC-Schicht porös sein soll.

Die Aufgabe wird gelöst durch ein Verfahren zum Herstellen einer dünnen Schicht aus porösem diamantähnlichem Kohlenstoff (DLC) mit plasmaunterstützter chemischer Gasphasenabscheidung (PECVD) entsprechend Anspruch 1, bei dem ein zu beschichtendes Werkstück in einem Rezipienten bereitgestellt und anschließend auf einer zu beschichtenden Oberfläche des Werkstücks eine dünne Schicht aus porösem DLC durch Ausführen der folgenden Schritte deponiert wird:
- Einleiten eines kohlenstoffhaltigen Precursor-Gases in den Rezipienten und Abscheiden einer DLC-Schicht auf der zu beschichtenden Oberfläche des Werkstücks bei einem Arbeitsdruck von 20^{∗}10⁻³ mbar bis 30^{∗}10⁻³ mbar und einer BIAS-Spannung von
- 250 V bis -150 V,
- anschließendes Einleiten eines Rückätz-Gases in den Rezipienten und Rückätzen der auf der Oberfläche des Werkstücks deponierten DLC-Schicht bei einem Arbeitsdruck von 200^{∗}10⁻³ mbar bis 300^{∗}10⁻³ mbar und einer BIAS-Spannung von -430 V bis -330 V.

Vorteilhaft weist die auf der Oberfläche des Werkstücks abgeschiedene DLC-Schicht, wenn sie mit den zuvor genannten Prozessparametern hergestellt wird, definierte Poren auf. Im Gegensatz zu plasmaunterstützt hergestellten DLC-Schichten herkömmlicher Art, die keine Poren aufweisen, eröffnen sich für die DLC-Schichten gemäß Aspekten der Erfindung ganz neue Anwendungsgebiete. Beispielsweise kommt eine solche DLC-Schicht als Diffusionsmembran mit definierter Permeabilität infrage. In der Medizintechnik kann sie als eine Barriere mit definierter und gezielt einstellbarer Diffusivität dienen. Sie kann beispielsweise für eine definierte Dauermedikamentation (controlled pharma release) eingesetzt werden. Hierzu wird beispielsweise die poröse DLC-Schicht auf eine Polymerschicht, welche ein Medikament umfasst, aufgebracht. Ein solches Schichtsystem kann beispielsweise auf der Oberfläche eines Implantats aufgebracht werden. Die DLC-Schicht ist eine biokompatible Oberfläche, welche gleichzeitig als kontrollierte und kontrollierbare Diffusionsbarriere wirkt.

In einem vollkommen anderen technischen Gebiet, wie beispielsweise dem Maschinenbau oder der Kraftfahrzeugtechnik, können die Poren einer porösen DLC-Schicht mit einem Schmiermittelreservoir versehen werden. Es wird eine Reibungs- und Verschleißminderung nicht nur durch die DLC-Schicht selbst, sondern zusätzlich durch das vorgehaltene Schmiermittel erreicht.

Die poröse DLC-Schicht weist gleichzeitig alle charakteristischen Eigenschaften und Vorteile auf, wie sie von herkömmlichen DLC-Schichten bekannt sind. Lediglich beispielshaft sei auf ihre gute Adhäsion auf einer Vielzahl von Werkstoffen, ihre große Härte und Verschleißfestigkeit sowie die Flexibilität des Herstellungsprozesses verwiesen.

Bei der Herstellung der porösen DLC-Schicht werden alternierend ein Depositionsschritt, in dem die DLC-Schicht auf der zu beschichtenden Oberfläche des Objekts mit den genannten Prozessparametern abgeschieden wird, und ein Rückätzschritt, bei dem die auf der Oberfläche des Werkstücks deponierte DLC-Schicht unter den genannten Prozessparametern zurückgeätzt wird, durchgeführt. Der Depositionsschritt und der Rückätzschritt werden so eingestellt, dass in der Summe eine positive Aufwachsrate erzielt wird. Es wird also mehr Material deponiert als zurückgeätzt. Es wurde erkannt, dass bei dem Rückätzschritt vor allem Schichtanteile mit einer SP2-Bindung (Graphitbindung) abgetragen werden. Bei den gewählten Prozessparametern ergibt sich selbstorganisiert eine poröse Oberfläche.

Der Depositionsschritt und der Rückätzschritt werden mehrfach wiederholt ausgeführt. Beispielsweise werden zwischen 2 und 25 Wiederholungen, beispielsweise 5 Wiederholungen, durchgeführt. Die poröse DLC-Schicht ist das Ergebnis des Depositionsschritts und des sich anschließenden Rückätzschritts, also beider Schritte.

Gemäß einer Ausführungsform ist das Verfahren dadurch fortgebildet, dass eine Depositionsrate, mit der die DLC-Schicht auf der zu beschichtenden Oberfläche deponiert wird, größer ist als eine Rückätzrate, mit der die auf der Oberfläche deponierte DLC-Schicht zurückgeätzt wird. Insbesondere ist vorgesehen, dass die Depositionsrate um mehr als einen Faktor fünf größer, insbesondere um mehr als einen Faktor zehn größer, insbesondere um mehr als einen Faktor 15 größer, und ferner insbesondere um höchstens einen Faktor 20 größer ist als die Rückätzrate.

Das Verhältnis der deponierten Menge und der rückgeätzen Menge, welche ebenso im Verhältnis der o.g. Faktoren zueinander stehen können, wird außer durch die Raten ferner beispielsweise durch die zeitliche Dauer der entsprechenden Schritte eingestellt. Ebenso ist vorgesehen, die Depositions- bzw. Rückätzrate durch Variation der Prozessparameter des Depositionsschritts bzw. des Rückätzschritts einzustellen. Die zuvor genannten Verhältnisse bzw. Faktoren zwischen Depositionsrate und Rückätzrate bzw. deponierter Menge und rückgeätzer Menge haben sich zur Herstellung einer porösen DLC-Schicht als besonders vorteilhaft erwiesen.

Ferner ist insbesondere vorgesehen, dass die Depositionsrate zwischen 100 nm/min und 160 nm/min liegt und die Rückätzrate zwischen 10 nm/min und 20 nm/min liegt.

Ebenso wie sich die zuvor genannten Verhältnisse zwischen der Depositionsrate und der Rückätzrate als vorteilhaft herausgestellt haben, haben sich auch die zuvor genannten absoluten Werte der Depositionsrate bzw. Rückätzrate in praktischen Versuchen als sehr geeignet erwiesen.

Gemäß einer weiteren Ausführungsform ist ferner vorgesehen, dass zwischen dem oder den Depositions- und Rückätzschritt(en) ein Relaxationsschritt durchgeführt wird, bei dem die Schicht einer Wärmebehandlung unterzogen wird.

Es ist ebenso vorgesehen, den Relaxationsschritt nicht zwischen dem Depositionsschritt und dem Rückätzschritt bzw. zwischen den Depositionsschritten und den Rückätzschritten durchzuführen, sondern die hergestellte poröse DLC-Schicht am Ende ihrer Herstellung einer Wärmebehandlung zu unterziehen.

Mit anderen Worten wird die Schicht im Relaxationsschritt ausgelagert bzw. angelassen. Es ist ebenso vorgesehen, einen Relaxationsschritt nach einer gewissen Anzahl von aufeinanderfolgenden Depositions- und Rückätzschritten durchzuführen. Dies kann einmal oder mehrfach während der Herstellung der porösen DLC-Schicht erfolgen.

Das Werkstück besteht zumindest an seiner zu beschichtenden Oberfläche zumindest überwiegend bevorzugt aus Titan oder Edelstahl. Insbesondere sind Edelstähle des Typs 316L oder X5CRNI vorgesehen. Ebenso sind weitere Materialien zur Herstellung einer darauf abgeschiedenen Schicht aus porösem DLC geeignet, beispielsweise Metalle oder auch Kunststoffe.

Mit den genannten Prozessparametern lassen sich mittlere Porengrößen bevorzugt zwischen 3 µm und 20 µm erzeugen. Die hergestellten deponierten Schichten zeigten eine exzellente Adhäsion und Abriebfestigkeit. Ihre Härte lag zwischen 5 GPa und 10 GPa. Diese Werte werden vielfach mit einem Nanoindenter gemessen. Die Adhäsion und Abriebfestigkeit wurde in Biegetests an verschiedenen Proben ermittelt. Außerdem wurde die hergestellte poröse DLC-Schicht mit Rasterelektronenmikroskop bzw. Transmissionselektronenmikroskopie sowie mit konfokaler Lasermikroskopie untersucht, insbesondere um die Porengröße und -verteilung zu bestimmen.

Für den Relaxationsschritt ist insbesondere vorgesehen, dass die Schicht bei einer Temperatur von weniger als 200°C für ein vorgegebenes Zeitintervall von insbesondere zwischen 15 min und 30 min relaxiert wird. Mit anderen Worten wird der Prozess, in welchem sich Deposition und Rückätzen stetig abwechseln, für eine bestimmte Zeitspanne unterbrochen. Der aktuell erreichte Zustand wird auf diese Weise gewissermaßen "eingefroren". Wenn die Herstellung anschließend fortgesetzt wird, geschieht dies mit anderer/veränderter Ausgangslage, so dass die Relaxationsschritte das erreichte Ergebnis, also die hergestellte poröse DLC-Schicht beeinflussen.

Gemäß einer weiteren Ausführungsform ist vorgesehen, dass das Precursor-Gas, insbesondere zumindest überwiegend, aus einem der folgenden Gase besteht: C₂H₂, CH₄ oder HMDSO (Hexamethyldisiloxan).

Ferner hat es sich als vorteilhaft herausgestellt, wenn das Rückätz-Gas zumindest überwiegend ein sauerstoffhaltiges Gas, insbesondere reiner Sauerstoff (O₂), und/oder ein fluorhaltiges Gas, insbesondere F₂ Trifluormethyl (CF₄ oder CF₃-Radikale) ist.

Auch die zuvor genannten Substanzen, welche als Precursor-Gas bzw. Rückätzgas zur Anwendung kommen, haben sich in praktischen Versuchen als besonders vorteilhaft erwiesen. Zusammenfassend können in dem Verfahren gemäß Aspekten der Erfindung poröse DLC-Schichten mit hervorragender Haftung, den gewünschten mechanischen Eigenschaften, beispielsweise im Hinblick auf das Elastizitätsmodul und Oberflächenspannung, mit gewünschter Porosität, Biokompatibilität und chemischer Zusammensetzung herstellt werden.

Die Aufgabe wird ferner gelöst durch die Verwendung einer PECVD-Anlage zum Durchführen eines Verfahrens gemäß einem oder mehreren der zuvor genannten Aspekte.

Ferner wird die Aufgabe gelöst durch ein Werkstück entsprechend Anspruch 10 mit einer Oberfläche, die mit einer porösen DLC-Schicht beschichtet ist, die in einem Verfahren nach einem oder mehreren der zuvor genannten Aspekte hergestellt ist.

Auf die Verwendung der PECVD-Anlage sowie auf das Werkstück treffen gleiche oder ähnliche Vorteile zu, wie sie bereits im Hinblick auf das Verfahren selbst erwähnt wurden. Sie sollen daher nicht wiederholt werden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte PECVD-Anlage,
- Fig. 2 und 3: rasterelektronenmikroskopische Aufnahmen einer porösen DLC-Schicht,
- Fig. 4: eine mit einem konfokalen Lasermikroskop durchgeführte Messung,
- Fig. 5: ein dieser Messung entnommenes Oberflächenprofil einer porösen DLC-Schicht und
- Fig. 6: ein Anwendungsbeispiel für ein Werkstück mit einer darauf vorhandenen porösen DLC-Schicht.

In den Zeichnungen sind jeweils gleiche oder gleichartige Elemente und/oder Teile mit denselben Bezugsziffern versehen, so dass von einer erneuten Vorstellung jeweils abgesehen wird.

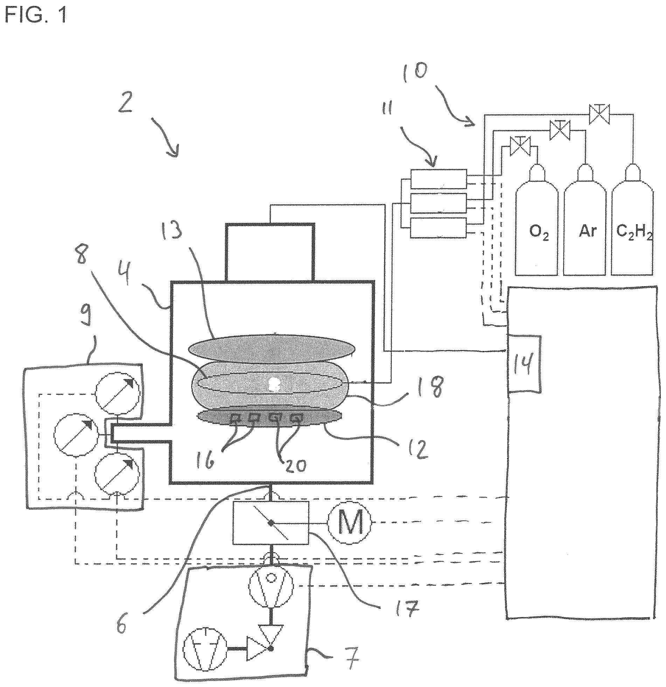

Fig. 1 zeigt in schematisch vereinfachter Ansicht eine PECVD-Anlage 2, deren Rezipient 4 über einen Anschlussflansch 6 an eine geeignete Pumpeneinheit 7 angeschlossen ist. Diese Pumpeneinheit 7 evakuiert den Innenraum des Rezipienten 4 bis auf einen Basisdruck von geringer als 10⁻³ mbar. Die Pumpeneinheit 7 umfasst beispielhaft eine Kombination auf einer Drehschieberpumpe und einer Turbomolekularpumpe. Zum Messen des Drucks im Inneren des Rezipienten 4 ist ein Drucksensor 9 an einem Flansch des Rezipienten 4 vorhanden. Der Drucksensor umfasst beispielhaft mehrere Druckmesser bzw. Druckmessdosen. Im Innenraum des Rezipienten 4 befindet sich ein Gaseinlass 8, beispielsweise ein mit Öffnungen versehener Ring, über den Prozessgas in den Innenraum des Rezipienten 4 eingeleitet wird. Hierzu ist der Gaseinlass 8 mit einer entsprechenden Prozessgasversorgung 10 verbunden. Die Prozessgase, beispielhaft sind C₂H₂ als Precursor-Gas, O₂ als Rückätzgas und Ar als Edelgas dargestellt, werden über jeweils einen Massestromregler 11 (flow controller) dem Innenraum des Rezipienten 4 über den Gaseinlass 8 zugeführt.

Ferner ist im Innenraum des Rezipienten 2 ein Probenhalter 12 angeordnet, der die Funktion einer Gegenelektrode erfüllt. Während der die Gegenelektrode auf Masse gelegt ist, ist eine RF-Elektrode 13 mit einer Wechselspannungsversorgung 14 gekoppelt. Auf dem Probenhalter 12 befinden sich die zu beschichtenden Werkstücke 16, vereinfacht als Substrate dargestellt. Die Werkstücke 16 umfassen eine oder mehrere zu beschichtende Oberflächen 20. Aus Gründen der Übersichtlichkeit sind lediglich einige Werkstücke 16 und Oberflächen 20 mit Bezugszeichen versehen.

Durch Herstellen der geeigneten Gasatmosphäre im Inneren des Rezipienten 4 und durch Anlegen der mittels der Wechselspannungsversorgung 14 bereitgestellten hochfrequenten Wechselspannung wird im Inneren des Rezipienten ein Plasma 18 erzeugt. Aus der Gasatmosphäre wird so eine Schicht aus diamantähnlichem Kohlenstoff (DLC) auf einer zu beschichtenden Oberfläche 20 der Werkstücke 16 abgeschieden. Es handelt sich in diesem Moment noch nicht um eine poröse DLC-Schicht.

Die Wechselspannungsversorgung 14 ist Teil einer Steuereinrichtung 15, durch die sie auch zum Zweck der Prozesssteuerung steuer- bzw. regelbar ist. Die Steuereinrichtung 15 dient ferner zum Ansteuern bzw. Auslesen der Pumpeneinheit 7, des Drucksensors 9 und der Massestromregler 11. Um den Arbeitsdruck im Rezipienten konstant zu halten steuert oder regelt die Steuereinrichtung 15 ferner ein Ventil 17 (Butterfly-Valve) über den Motor M.

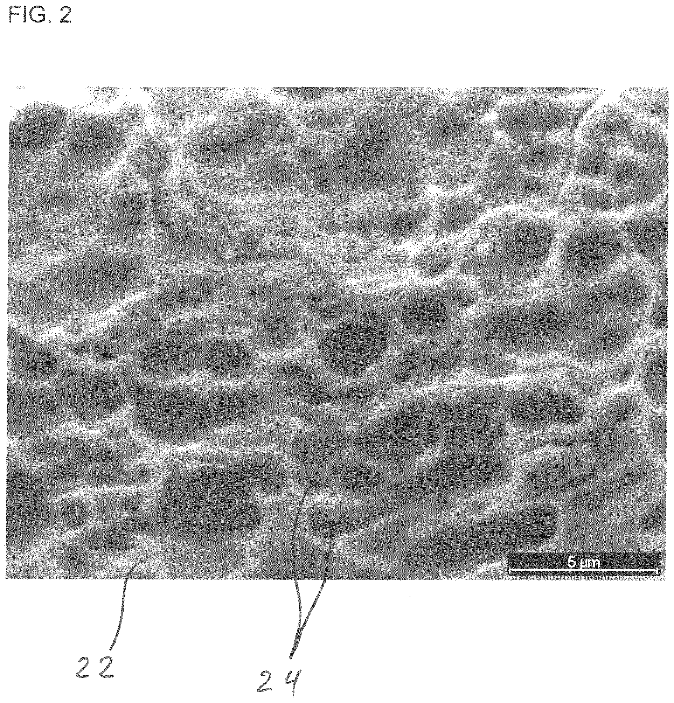

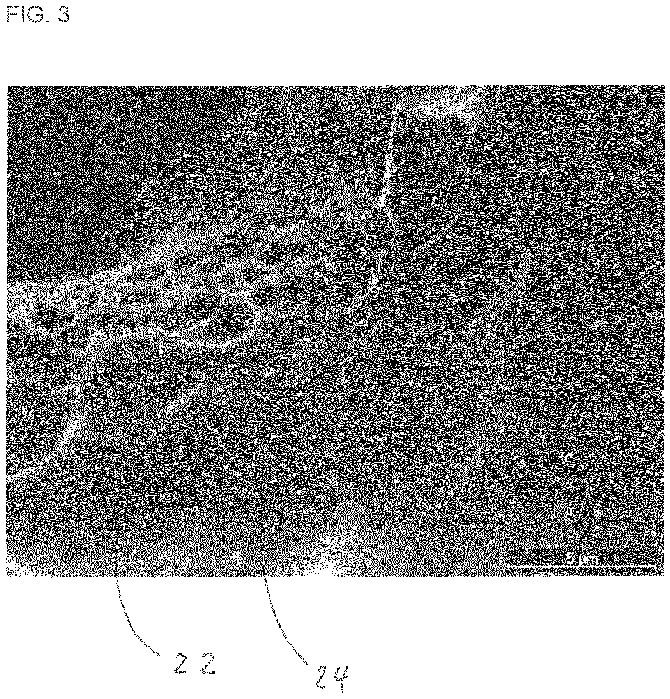

Die Fig. 2 und 3 zeigen rasterelektronenmikroskopische Aufnahmen einer porösen DLC-Schicht 22, wie sie auf einem Werkstück in der gezeigten Anlage herstellbar sind. Deutlich sichtbar sind die Poren 24 unterschiedlicher Größe.

Zum Herstellen der porösen DLC-Schicht 22 auf der Oberfläche 20 des Werkstücks 16 wird das Werkstück 16 zunächst in den Rezipienten 4 verbracht, welcher anschließend auf den gewünschten Basisdruck evakuiert wird. Das Werkstück 16 ist beispielsweise aus Titan oder Edelstahl, beispielsweise aus Edelstahl des Typs 316L oder X5CRNI. Über die Massestromregler 11 wird anschließend Prozessgas dem Rezipienten 4 zugeführt. Als Prozessgase sind ferner beispielsweise C₂H₂, CH₄ oder HMDSO (Hexamethyldisiloxan) geeignet. Das Prozessgas wird beispielsweise mit einer Flussrate von 5 sccm dem Rezipienten 4 zugeführt.

Mit Hilfe der Wechselspannungsversorgung 14 wird anschließend eine hochfrequente Wechselspannung beispielsweise mit einer Frequenz von 13,56 MHz angelegt. Nach dem Zünden des Plasmas 18 stellt sich eine BIAS-Spannung (self-BIAS) von beispielsweise -200 V ein und auf der Oberfläche 20 des Werkstücks 16 wird eine DLC-Schicht abgeschieden. Dies geschieht bei einem Arbeitsdruck zwischen 20^{∗}10⁻³ mbar und 30^{∗}10⁻³ mbar. Die BIAS-Spannung liegt zwischen -150 V und -250 V und liegt zwischen der RF-Elektrode 13 und der aus Masse liegenden Gegenelektrode (Probenhalten 12) an. Ebenso wie der zuvor genannte Arbeitsdruck kann auch die BIAS-Spannung im angegebenen Intervall liegen.

Die auf der Oberfläche 20 des Werkstücks 16 abgeschiedene Schicht ist zunächst noch keine poröse DLC-Schicht. Die Porosität der Schicht wird erst erreicht, indem in einem sich anschließenden Rückätzschritt ein Teil der deponierten DLC-Schicht wieder entfernt wird und der Depositionsschritt und der Rückätzschritt mehrfach wiederholt werden. Der Rückätzschritt erfolgt, indem nach zumindest überwiegendem Entfernen des Prozessgases aus dem Rezipienten 4 und Beendigung der Plasmaentladung ein Rückätzgas in den Rezipienten 4 eingeleitet wird. Dies erfolgt erneut über den Gaseinlass 8 und eine entsprechende Regelung durch die Massestromregler 11.

Als Rückätzgas ist insbesondere ein sauerstoffhaltiges Gas, beispielsweise reiner Sauerstoff O₂, oder auch ein fluorhaltiges Gas, beispielsweise F₂ oder Trifluormethyl, geeignet. Das Rückätzgas wird beispielsweise mit einer Flussrate von 90 sccm dem Rezipienten 4 zugeführt. Der Rückätzschritt erfolgt bei einem Arbeitsdruck zwischen 200^{∗}10⁻³ mbar und 300^{∗}10⁻³ mbar und einer BIAS-Spannung zwischen -330 V und -340 V, beispielsweise von -380 V. Erneut kann die BIAS-Spannung ebenso wie der Arbeitsdruck im zuvor genannten Intervall liegen.

Es hat sich herausgestellt, dass während des Rückätzschritts bevorzugt die SP2-Anteile der deponierten DLC-Schicht entfernt werden. Dieser selektive Ätzprozess konnte neben ebenfalls stattfindenden Selbstorganisationsprozessen als wesentlicher Mechanismus für das Entstehen der Poren 24 in der porösen DLC-Schicht 22 identifiziert werden. Je nach gewünschter Gesamtschichtdicke erfolgen anschließend erneut ein Depositionsschritt sowie ein zugehöriger Rückätzschritt. Dieses Verfahren wird so lange wiederholt, bis die gewünschte Schichtdicke der porösen DLC-Schicht 22 auf der Oberfläche 20 des Werkstücks 16 erreicht ist.

Sowohl für den Depositionsschritt als auch für den Rückätzschritt kann eine gemischte Atmosphäre verwendet werden, der neben dem Prozess- bzw. Rückätzgas eine Edelgaskomponente, beispielsweise Argon, beigemischt wird.

Um ein ausreichendes Schichtwachstum auf der Oberfläche 20 des Werkstücks 16 zu erreichen, ist vorgesehen, dass die DLC-Schicht auf der zu beschichtenden Oberfläche 20 mit einer Depositionsrate abgeschieden wird, die größer ist als eine Rückätzrate, mit der die auf der Oberfläche deponierte DLC-Schicht zurückgeätzt wird. Diese Angaben beziehen sich auf die DLC-Schicht und nicht auf die poröse DLC-Schicht, welche im Zusammenwirken von Deposition und Rückätzen erst entsteht.

Es hat sich als vorteilhaft herausgestellt, wenn die Depositionsrate bzw. die deponierte Menge um ein Vielfaches größer ist als die Rückätzrate bzw. die rückgeätze Menge. Geeignete Faktoren sind beispielsweise 3, 5, 7, 10, 13, 15, 17 und 20. Höhere Rückätzraten sind gemäß einem Ausführungsbeispiel nicht vorgesehen. Mit anderen Worten beträgt also die Depositionsrate insbesondere maximal das Zwanzigfache der Rückätzrate.

Ferner hat es sich als vorteilhaft erwiesen, wenn die Depositionsrate zwischen 100 nm/min und 160 nm/min, insbesondere zwischen 110 nm/min und 150 nm/min, ferner insbesondere zwischen 120 nm/min und 140 nm/min liegt. Mit anderen Worten sind also beispielsweise Depositionsraten von 100 nm/min, 110 nm/min, 120 nm/min, 130 nm/min, 140 nm/min oder 150 nm/min vorgesehen. Ferner hat es sich als vorteilhaft herausgestellt, wenn die Rückätzrate zwischen 10 nm/min und 20 nm/min, insbesondere zwischen 12 nm/min und 18 nm/min, ferner insbesondere zwischen 14 nm/min und 16 nm/min liegt. Mit anderen Worten sind also insbesondere Rückätzraten von 10 nm/min, 12 nm/min, 14 nm/min, 16 nm/min, 18 nm/min oder 20 nm/min vorgesehen.

Die poröse DLC-Schicht 22 entsteht bei den angegebenen Prozessparametern durch Selbstorganisationprozesse auf der zu beschichtenden Oberfläche 20. Es hat sich ferner als vorteilhaft herausgestellt, wenn in die Herstellung Relaxationsschritte integriert werden, bei denen die poröse DLC-Schicht 22 relaxiert wird. Mit anderen Worten wird also die Herstellung der porösen DLC-Schicht 22 kurzfristig angehalten und ausgehend von dem zwischenzeitlich "eingefrorenen" Zustand anschließend fortgesetzt. Die gesamte Herstellung erfolgt beispielsweise bei Temperaturen von weniger als 200°C. Ein geeignetes Zeitintervall für den Relaxationsschritt liegt zwischen 15 min und 30 min. Der Relaxationsschritt hat also eine Dauer von beispielsweise 5, 10, 20, 25 oder 30 min.

Der Relaxationsschritt kann zwischen dem Depositions- und dem Rückätzschritt erfolgen, wobei zwischen jedem oder lediglich einigen der Depositions- und Rückätzschritten ein Relaxationsschritt vorgesehen wird.

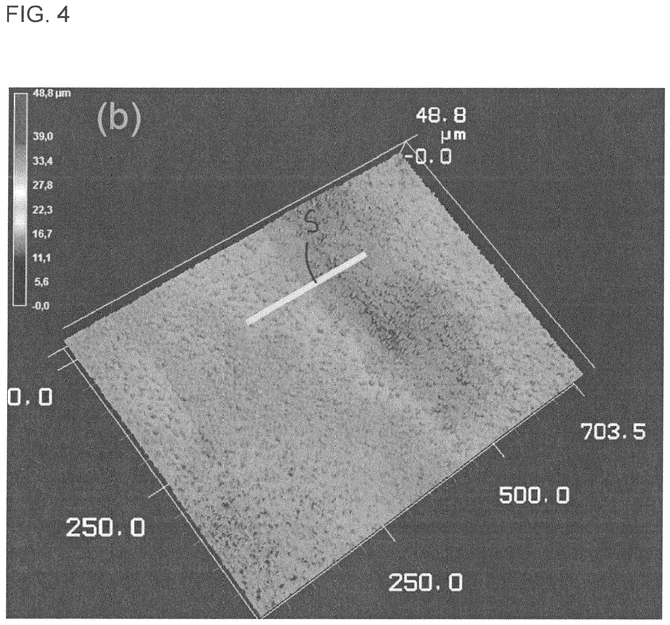

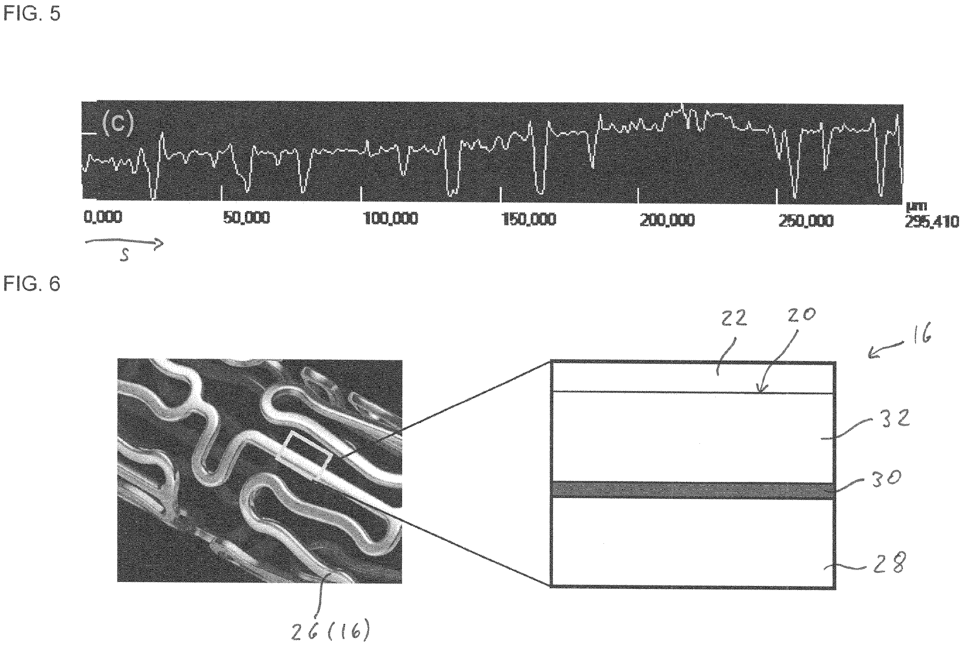

Fig. 4 zeigt eine Messung, welche mit konfokaler Lasermikroskopie durchgeführt wurde. Sichtbar ist eine Oberfläche 20 einer porösen DLC-Schicht 22. Entlang der Strecke S ist eine Profilmessung der Oberflächenmorphologie der hergestellten porösen DLC-Schicht 22 durchgeführt worden. Das Ergebnis einer solchen beispielhaften Messung entlang der Strecke S zeigt Fig. 5.

In Fig. 5 ist die Messstrecke S auf der Abszisse und die Höhe der Oberfläche in beliebigen Einheiten auf der Ordinate aufgetragen. Die ausgeprägten Minima des Profils zeigen die in der porösen DLC-Schicht 22 vorhandenen Poren 24 an. Aus Messungen mit einem Nanoindenter wurde eine mittlere Porengröße ermittelt, welche beispielsweise zwischen 3 µm und 20 µm liegt. Biege- und Abrasionstests haben außerdem ergeben, dass die poröse DLC-Schicht 22 eine ausgezeichnete Haftung und eine große Abrasionsfestigkeit zeigt. Die Härte der porösen DLC-Schicht 22 liegt zwischen 5 und 10 GPa. Diese Werte wurden ebenfalls mit einem Nanoindenter ermittelt.

Es hat sich ferner herausgestellt, dass die Porengröße sehr gut über die Größe der BIAS-Spannung kontrollierbar ist. Dabei skaliert die mittlere Porengröße zumindest näherungsweise proportional mit der BIAS-Spannung.

Fig. 6 zeigt in einer vereinfachten Darstellung ein Detail eines Stents 26 als Beispiel für ein Werkstück 16. Die schematisch dargestellte Detail- und Querschnittsansicht durch einen Steg des Stents 26 in Fig. 6 zeigt, dass dieser für eine kontrollierte Medikamentenabgabe über einen längeren Zeitraum eine spezielle Schichtstruktur aufweist. Auf einem Basismaterial 28, beispielsweise Titan oder Edelstahl, ist zunächst ein Haftvermittler 30 aufgebracht. Auf diesem befindet sich eine Polymerschicht 32, welche mit einem Medikament versetzt ist. Auf deren Oberfläche befindet sich eine poröse DLC-Schicht 22, welche eine Diffusionsbarriere mit definierter Diffusivität darstellt. Die Durchlässigkeit der porösen DLC-Schicht 22 ist unter anderem von der mittleren Porengröße der in der porösen DLC-Schicht 22 vorhandenen Poren 24 abhängig. So kann eingestellt werden, mit welcher Rate das in der Polymerschicht 32 vorhandene Medikament über die poröse DLC-Schicht 22 in die Umgebung abgegeben wird.

Ein weiteres Ausführungsbeispiel ist ein Werkstück 16 im Bereich des Maschinenbaus, welches nicht dargestellt ist. Es ist ähnlich aufgebaut, wie das in Fig. 6 gezeigte Werkstück 16. Auf einem Basismaterial 28, beispielsweise Stahl, wird direkt eine poröse DLC-Schicht 22 aufgebracht. Diese DLC-Schicht dient beispielsweise der Verminderung der Reibung. Bei dem Werkstuck kann es sich um eine Nockenwelle und bei der Oberfläche um deren Steuerfläche handeln. Die Poren 24 der porösen DLC-Schicht 20 können mit einem reibungsvermindernden Zusatz oder Schmiermittel versehen werden. Die DLC-Schicht 22 wirkt durch das kontinuierlich hinzudosierte Schmiermittel reibungsvermindernd.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: PECVD-Anlage
- 4: Rezipient
- 6: Anschlussflansch
- 7: Pumpeneinheit
- 8: Gaseinlass
- 9: Drucksensor
- 10: Prozessgasversorgung
- 11: Massestromregler
- 12: Probenhalter
- 13: RF-Elektrode
- 14: Hochspannungsversorgung
- 15: Steuereinrichtung
- 16: Werkstück
- 17: Ventil
- 18: Plasma
- 20: Oberfläche
- 22: poröse DLC-Schicht
- 24: Poren
- 26: Stent
- 28: Basismaterial
- 30: Haftvermittler
- 32: Polymerschicht
- M: Motor

## Patentansprüche

1. Verfahren zum Herstellen einer dünnen Schicht (22) aus porösem diamantähnlichem Kohlenstoff (DLC) mit plasmaunterstützter chemischer Gasphasenabscheidung (PECVD), bei dem ein zu beschichtendes Werkstück (16) in einem Rezipienten (4) bereitgestellt und anschließend auf einer zu beschichtenden Oberfläche (20) des Werkstücks (16) eine dünne Schicht aus porösem DLC durch mehrfach wiederholtes Ausführen der folgenden Schritte deponiert wird:
Einleiten eines kohlenstoffhaltigen Precursor-Gases in den Rezipienten (4) und Abscheiden einer DLC-Schicht auf der zu beschichtenden Oberfläche (20) des Werkstücks (16) bei einem Arbeitsdruck von 20^{∗}10⁻³ mbar bis 30^{∗}10⁻³ mbar und einer BIAS-Spannung von -250 V bis -150 V,
anschließendes Einleiten eines Rückätz-Gases in den Rezipienten (4) und Rückätzen der auf der Oberfläche (20) des Werkstücks (16) deponierten DLC-Schicht bei einem Arbeitsdruck von 200^{∗}10⁻³ mbar bis 300^{∗}10⁻³ mbar und einer BIAS-Spannung von -430 V bis -330 V.

2. Verfahren nach Anspruch 1, bei dem eine Depositionsrate, mit der die DLC-Schicht auf der zu beschichtenden Oberfläche (20) deponiert wird, größer ist als eine Rückätzrate, mit der die auf der Oberfläche (20) deponierte DLC-Schicht zurückgeätzt wird.

3. Verfahren nach Anspruch 2, bei dem die Depositionsrate um mehr als einen Faktor fünf größer, insbesondere um mehr als einen Faktor zehn größer, insbesondere um mehr als einen Faktor 15 größer, und ferner insbesondere um höchstens einen Faktor 20 größer ist als die Rückätzrate.

4. Verfahren nach Anspruch 2 oder 3, bei dem die Depositionsrate zwischen 100 nm/min und 160 nm/min liegt und die Rückätzrate zwischen 10 nm/min und 20 nm/min liegt.

5. Verfahren nach einem der vorhergehenden Ansprüche, bei dem, nachdem zwischen dem oder den Depositions- und Rückätzschritt(en) ein Relaxationsschritt durchgeführt wird.

6. Verfahren nach Anspruch 5, bei dem in dem Relaxationsschritt die Schicht bei einer Temperatur von kleiner als 200°C für ein vorgegebenes Zeitintervall von insbesondere zwischen 15 min und 30 min relaxiert wird.

7. Verfahren nach einem der vorhergehenden Ansprüche, wobei das Precursor-Gas, insbesondere zumindest überwiegend, aus einem der folgenden Gase besteht: C₂H₂, CH₄ oder HMDSO (Hexamethyldisiloxan).

8. Verfahren nach einem der vorhergehenden Ansprüche, wobei das Rückätz-Gas zumindest überwiegend ein sauerstoffhaltiges Gas, insbesondere reiner Sauerstoff (O₂), und/oder ein fluorhaltiges Gas, insbesondere F₂ oder Trifluormethyl, ist.

9. Verwenden einer PECVD-Anlage (2) zum Durchführen eines Verfahrens nach einem der vorhergehenden Ansprüche.

10. Werkstück (16) mit einer Oberfläche (20), die mit einer porösen DLC-Schicht (22) beschichtet ist, die in einem Verfahren nach einem der Ansprüche 1 bis 8 hergestellt ist und **dadurch gekennzeichnet ist, dass** die mittlere Porengröße zwischen 3 µm und 20 µm beträgt.

## Claims

1. A method of producing a thin layer (22) of a porous, diamond-like carbon (DLC) by plasma-enhanced chemical vapor deposition (PECVD) in which a workpiece (16) to be coated is provided in a recipient (4), and then a thin layer of porous DLC is deposited on a surface (20) of the workpiece (16) to be coated by repeatedly performing the following steps:
- feeding a carbon-containing precursor gas into the recipient (4), and depositing a DLC layer on the surface (20) of the workpiece (16) to be coated at an operating pressure of 20^{∗}10⁻³ mbar to 30^{∗}10⁻³ mbar and a BIAS voltage of -250 V to -150 V,
- then feeding a back-etching gas into the recipient (4), and depositing a DLC layer on the surface (20) of the workpiece (16) at an operating pressure of 200^{∗}10⁻³ mbar to 300^{∗}10⁻³ mbar and a BIAS voltage of -430 V to -330 V.

2. The method according to claim 1, wherein a deposition rate with which the DLC layer is deposited on the surface (20) to be coated is greater than a back-etching rate with which the DLC layer deposited on the surface (20) is etched back.

3. The method according to claim 2, wherein the deposition rate is greater by more than a factor of five, in particular greater by more than a factor of 10, in particular greater by more than a factor of 15 and furthermore in particular at most greater by factor of 20 than the back-etching rate.

4. The method according to claim 2 or 3, wherein the deposition rate is between 100 nm/min and 160 nm/min, and the back etching rate is between 10 nm/min and 20 nm/min.

5. The method according to one of the preceding claims, wherein a relaxation step is performed between the deposition and back etching step(s).

6. The method according to claim 5, wherein the layer is relaxed at a temperature of less than 200°C for a given interval of time of in particular between 15 minutes and 30 minutes during the relaxation step.

7. The method according to one of the preceding claims, wherein the precursor gas consists at least predominantly of one of the following gases: C₂H₂, CH₄ or HMDSO (hexamethyldisiloxane).

8. The method according to one of the preceding claims, wherein the back-etching gas is at least predominantly an oxygen-containing gas, in particular pure oxygen (O₂), and/or a fluorine-containing gas, in particular F₂ or trifluoromethyl.

9. A use of a PECVD system (2) to perform a method according to one of the preceding claims.

10. A workpiece (16) with the surface (20) that is coated with a porous DLC layer (22) which is produced by the method according to one of claims 1 to 8 and is **characterized in that** the average pore size is between 3 µm and 20 µm.

## Revendications

1. Procédé pour former une couche mince (22) constituée de carbone poreux de type diamant (DLC) par dépôt chimique en phase vapeur assisté par plasma (PECVD), dans lequel une pièce (16) devant être revêtue est fournie dans un récipient (4) et une couche mince de DLC poreux est ensuite déposée sur une surface (20) de la pièce (16) à revêtir, en exécutant plusieurs fois les étapes suivantes consistant à :
introduire un gaz de précurseur contenant du carbone dans le récipient (4) et déposer une couche de DLC sur la surface (20) de la pièce (16) à revêtir à une pression de travail de 20^{∗}10⁻³ mbar à 30^{∗}10⁻³ mbar et à une tension de polarisation de - 250 V à - 150 V,
introduire ensuite un gaz de rétrogravure dans le récipient (4) et rétrograver la couche de DLC déposée sur la surface (20) de la pièce (16) à une pression de travail de 200^{∗}10⁻³ mbar à 300^{∗}10⁻³ mbar et à une tension de polarisation de - 430 V à - 330 V.

2. Procédé selon la revendication 1, dans lequel une vitesse de dépôt à laquelle la couche de DLC est déposée sur la surface (20) à revêtir, est supérieure à une vitesse de rétrogravure à laquelle la couche de DLC déposée sur la surface (20) est rétrogravée.

3. Procédé selon la revendication 2, dans lequel la vitesse de dépôt est supérieure à la vitesse de rétrogravure d'un facteur supérieur à 5, en particulier d'un facteur supérieur à 10, en particulier d'un facteur supérieur à 15 et, de plus, en particulier d'un facteur au plus égal à 20.

4. Procédé selon la revendication 2 ou 3, dans lequel la vitesse de dépôt est comprise entre 100 nm/min et 160 nm/min et la vitesse de rétrogravure est comprise entre 10 nm/min et 20 nm/min.

5. Procédé selon l'une des revendications précédentes, dans lequel une étape de relaxation est exécutée entre l'étape (ou les étapes) de dépôt et de rétrogravure.

6. Procédé selon la revendication 5, dans lequel, à l'étape de relaxation, la couche est relaxée à une température inférieure à 200 °C pendant un intervalle de temps prédéfini, en particulier compris entre 15 min et 30 min.

7. Procédé selon l'une des revendications précédentes, dans lequel le gaz de précurseur est constitué, notamment au moins en majeure partie, de l'un des gaz suivants : C₂H₂, CH₄ ou HMDSO (hexaméthyldisiloxane).

8. Procédé selon l'une des revendications précédentes, dans lequel le gaz de rétrogravure est au moins majoritairement un gaz contenant de l'oxygène, en particulier de l'oxygène pur (0₂), et/ou un gaz contenant du fluor, en particulier F₂ ou du trifluorométhyle.

9. Utilisation d'une installation de PECVD (2) pour mettre en oeuvre un procédé selon l'une des revendications précédentes.

10. Pièce (16) ayant une surface (20) revêtue d'une couche de DCL poreuse (22) formée par un procédé selon l'une des revendications 1 à 8 et **caractérisée en ce que** la taille de pores moyenne est comprise entre 3 µm et 20 µm.
